# EUROPEAN PATENT SPECIFICATION

(11) **EP 2 099 431 B1**
(45) Date of publication and mention of the grant of the patent: **05.06.2013**
(21) Application number: 07847376.6
(22) Date of filing: 27.11.2007
(51) Int. Cl.: A61K 9/20, A61K 31/4184

(54) **Stabilized solid pharmaceutical composition of candesartan cilexetil**
Stabilisierte feste pharmazeutische zusammensetzung von candesartan cilexetil
Composition pharmaceutique solide stabilisee de candesartan cilexetil

(30) Priority: 28.11.2006 ES 200603089; 25.01.2007 US 886608 P
(43) Date of publication of application: 16.09.2009
(73) Proprietor: Laboratorios Liconsa, S.A., 08028 Barcelona (ES)
(72) Inventor: JURADO SÁNCHEZ, Francisco, 19200 Azuqueca De Henares (Guadalajara) (ES); MORENO RUEDA, Juan, 08849 Sant Climent De Llobregat (Barcelona) (ES); JOHANSSON, Mona, 08017 Barcelona (ES); ARROYO HIDALGO, Sergio, 28011 Madrid (ES); DEL CURA MEDINA, Estíbaliz, 28043 Madrid (ES); CONDE AGUILAR, Susana, 28050 Madrid (ES)
(74) Representative: ZBM Patents
(86) International application number: PCT/EP2007/062849
(87) International publication number: WO 2008/065097

(56) References cited:
- EP-A- 1 629 835
- WO-A-01/37808
- WO-A-2005/034920
- WO-A-2005/041929
- WO-A-2005/070398
- WO-A-2005/079751
- WO-A-2006/074218
- WO-A-2006/084475
- WO-A-2006/113631
- US-A1- 2003 180 352
- US-A1- 2006 024 361
- US-A1- 2006 034 937

## Description

### Field of the invention

The present invention relates to solid pharmaceutical compositions comprising candesartan cilexetil, which exhibit a high stability of the active substance. The invention also relates to the use thereof in the preparation of solid oral dosage forms of candesartan cilexetil.

### Background of the invention

Candesartan cilexetil is the INN (International Nonproprietary Name) of 2-ethoxy-1-[[2'-(1H-tetrazol-5-yl)[1,1'-biphenyl]-4-yl]methyl]-1H-benzimidazole-7-carboxylic acid, 1-[[(cyclohexyloxy)carbonyl]oxy]ethyl ester having the following structural formula:

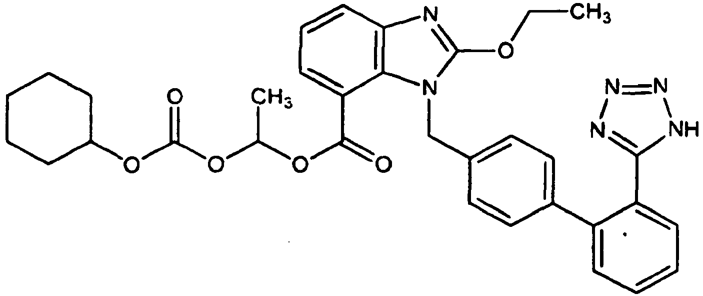

Candesartan cilexetil is an active ingredient, which was disclosed for the first time in European Patent Application EP-A-0459136. Due to its capacity in acting as an angiotensin II receptor antagonist, it is used as an antihypertensive agent.

Patent Application EP-A-0546358 discloses that solid compositions including an angiotensin II receptor antagonist as active ingredient, for example candesartan cilexetil, show a reduction in the content of active ingredient with the lapse of time due to a crystal deformation caused, for example, by the pressure, abrasion or heat applied during the granulation or compression process.

It is also described in EP-A-0546358 that these compositions show higher stability if an oily substance selected from the group consisting of hydrocarbons, fatty acids, long-chain alcohol, esters of a fatty acid and a polyhydroxy alcohol, ethers of a long-chain alcohol and a polyhydroxy alcohol, and alkylene oxide polymers or copolymers, is incorporated into the formulation.

Patent Application WO-A-2005/070398 discloses that the incorporation of cosolvents into the formulations of candesartan cilexetil also contributes to the stabilization of this active substance. Among cosolvents, propylenglycol, polyethylenglycol, ethanol, glycerol, propylenglycol esters and polyethylenglycol esters are described.

Patent Application WO-A-2005/079751 discloses another technical solution for improving stability of candesartan cilexetil compositions which consists of the incorporation of lipids and phospholipids. Among lipids, fatty acids and fatty acid esters, particularly, fatty acid glycerol esters are described. Among phospholipids, phosphoglycerides and sphyngolipids are described.

Patent Application WO-A-2005/084648 discloses pharmaceutical compositions of candesartan cilexetil, which are stabilized by hydrosoluble polymers, for example, xanthan gum, polyvinyl alcohol, and maltodextrin. Further relevant prior art includes EP 1468683.

Therefore, there is a need to provide alternative pharmaceutical compositions of candesartan cilexetil which exhibit a good stability of the active substance.

On the basis of numerous studies, the present inventors have discovered a group of stabilizing agents that allow the preparation of pharmaceutical compositions of candesartan cilexetil, wherein the active substance exhibits good stability over time in spite of the fact that it has been exposed to granulation and/or compression process.

### Summary of the invention

The object of the present invention is a solid pharmaceutical composition comprising candesartan cilexetil, as an active substance, and a stabilizing agent.

The invention encompasses a process for the preparation of this solid pharmaceutical composition.

Also provided is the use of this composition for the preparation of solid oral dosage forms of candesartan cilexetil.

Another aspect of the invention relates to a tablet containing candesartan cilexetil, which comprises the pharmaceutical composition of the invention.

### Description of the invention

The object of the invention is a solid pharmaceutical composition for oral use comprising:
a) a pharmaceutically effective amount of candesartan cilexetil, and
b) a stabilizing agent selected from the group consisting of :
   - ethers of C₁-C₄ monohydroxy alcohols and C₂-C₉ polyhydroxy alcohols,
   - saturated fatty acid alkaline salts, and
   - panthenol.

### Active substance

The active substance of the composition of this invention is candesartan cilexetil, which may be prepared, for instance, in accordance with the process disclosed in Patent Application EP-A-0459136.

In the composition of the invention, any crystalline or amorphous form of the active ingredient may be used. Preferably, a stable crystalline form as described in Experimental Example 1 of EP-A-0459136 is used.

### Stabilizing agent

The stabilizing agent used according to the present invention is selected from the group consisting of, ethers of C₁-C₄ monohydroxy alcohols and C₂-C₉ polyhydroxy alcohols, saturated fatty acid alkaline salts, and panthenol.

Mixtures of these stabilizing agents may also be used in the composition of the invention.

For the purposes of the invention, the term "saturated fatty acids" refers to those fatty acids that have a carbon-atom chain equal to or higher than C₆, that do not have unsaturated double or triple bonds along the hydrocarbon chain, and have only one carboxylic group. These fatty acids may be, for instance, caproic acid (C₆), caprilic acid (C₈), capric acid (C₁₀), lauric acid (C₁₂), myristic acid (C₁₄), palmitic acid (C₁₆), stearic acid (C₁₈), arachidic acid (C₂₀), behenic acid (C₂₂), and lignoceric acid (C₂₄). Also included are mixtures of saturated carboxylic acids derived from splitting hydrogenation of oils and natural fats, for example, hydrogenated coconut fatty acids, hydrogenated palm fatty acids, hydrogenated rapeseed fatty acids, hydrogenated tallow fatty acids, and hydrogenated castor oil fatty acids. Also included are those carboxylic acids that have a carbon-atom chain equal to or higher than C₆ and wherein the hydrocarbonated chain is branched, for example, 2-ethylhexanoic acid (branched C₈), and isostearic acid (branched C₁₈).

Monohydroxy alcohols are those alcohols which have only one hydroxyl group. They include methanol, ethanol, n-propanol, n-butanol, n-penthanol, lauryl alcohol (C₁₂), myristyl alcohol (C₁₄), palmyl alcohol (C₁₆), stearyl alcohol (C₁₈), behenyl alcohol (C₂₂). In the context of the present invention, the term "monohydroxy alcohols" also refers to those alcohols wherein the hydrocarbonated chain is branched, such as isopropanol, isobutanol, sec-butanol, 2-ethylhexanol (branched C₈), isododecyl alcohol (branched C₁₂), isotridecyl alcohol (branched C₁₃), isostearyl alcohol (branched C₁₈), and alcohol 2-octyldodecyl (branched C₂₀).

Hydroxycarboxylic acids are carboxylic or polycarboxylic acids that include one or more hydroxyl groups in their structure, such as, glycolic acid (hydroxyethanoic acid), lactic acid (2-hydroxypropanoic acid), glyceric acid (2,3-dihydroxypropanoic acid), tartronic acid (hydroxypropanedioic acid), malic acid (hydroxybutanedioic acid), and citric acid (2-hydroxy-1,2,3-propanetricarboxylic acid).

In the context of the present invention, the term "C₂-C₉ polyhydroxy alcohols" are those alcohols that have two or more hydroxyl groups in their molecule, for example, ethylenglycol, diethylenglycol, triethylenglycol, propylenglycol, dipropylenglycol, glycerin, diglycerin, triglycerin, sorbitol and sorbitan.

Saturated fatty acid alkaline salts, also denominated soaps, are constituted by a carboxylate anion derived from the saturated fatty acid chain an by an alkali metal cation, for example, lithium, sodium or potassium. Among others, sodium palmitate, sodium stearate, potassium stearate, potassium palmitate, and sodium myristate may be mentioned.

Panthenol is a common name for 2,4-dihydroxy-N-(3-hydroxypropyl)-3,3-dimethylbutanamide, which is also denominated provitamin B5. The compound has a chiral centre, and both the racemic form (DL-panthenol) and optically active D-panthenol are commercially available. Either of them is suitable to be used in the composition of the invention as a stabilizing agent of candesartan cilexetil.

Esters of saturated fatty acids and monohydroxy alcohols include, for example, isopropyl myristate, isopropyl palmitate, isopropyl stearate, n-butyl stearate, 2-ethylexyl palmitate, 2-ethylexyl stearate, 2-octyldodecyl myristate, 2-ethylhexyl isostearate, isostearyl isostearate, isononyl isononanoate. Esters of this type are commercially available, for example, under the trade names PRISORINE^{®} and PRIOLUBE^{®} from Uniqema, or WAGLINOL^{®} and LASEMUL^{®} from Industrial Quimica Lasem.

In general, the esters denominated stearates contain a mixture of C₁₆ (palmitic) acid and C₁₈ (stearic) acid, wherein the latter is the major component, even though other stearates containing approximately 100% stearic acid are also commercially available.

Suitable ethers of C₁-C₄ monohydroxy alcohols and C₂-C₉ polyhydroxy alcohols may include, for example, diethylenglycol monoethyl ether, ethylenglycol monobutyl ether, diethylenglycol monobutyl ether, triethylenglycol monobutyl ether, triethylenglycol monoethyl, ethylenglycol monomethyl ether, and diethylenglycol mono-n-propyl ether. These ethers are commercially available, for example, under the trade names DOWANOL^{®}, CELLOSOLVE^{®}, and CARBITOL^{®} from Dow.

Preferably the stabilizing agent is selected from esters of hydroxycarboxylic acids and C₁-C₄ monohydroxy alcohols, and ethers of C₁-C₄ monohydroxy alcohols and C₂-C₉ polyhydroxy alcohols.

Preferably the stabilizing agent is ether of a C₁-C₄ monohydroxy alcohol and a C₂-C₉ polyhydroxy alcohol, more preferably diethylenglycol ethyl ether.

### Preparation process

The invention also encompasses a process for the preparation of a solid pharmaceutical composition of candesartan cilexetil, which comprises mixing the active substance with the stabilizing agent and moulding the mixture.

Preferably the mixture is moulded by granulation or high-pressure compression, so that granules or tablets can be prepared.

For example, if the stabilizing agent is a solid product it can be directly added to the active substance, and the mixture may be moulded by compression. A solvent may also be added, and performing a granulation step and subsequent drying. If the stabilizing agent is a liquid product it can also be sprayed directly onto the active substance followed by kneading and granulation. If the stabilizing agent is a product wherein the melting point is in a range of approximately 30°C to 90°C it can be added in the melted state. A solution or dispersion of the stabilizing agent and the active substance may be each prepared and may be sprayed onto a pulverulent solid inert excipient, and then the composition may be conventionally kneaded, granulated and dried. In case a solvent is used, the solvent should not exert any harmful effect on the active substance, such as for example water, ethanol, isopropyl alcohol, methylene chloride, or dimethylformamide.

The incorporation of the stabilizing agent into the solid composition of the invention allows stabilization of the active substance in the kneading, granulation and compression process.

As it is known by those skilled in the art, different auxiliary agents may be used for preparing the solid compositions of the invention, which may be added during the appropriate phase so as to afford the appropriate mechanical and release properties. Preferably the auxiliary agent according to the present invention is used during the moulding phase.

### Solid dosage forms for oral administration

The invention encompasses the use of the compositions of the invention for the preparation of solid dosage forms of candesartan cilexetil for oral administration.

Preferably the solid dosage forms of candesartan cilexetil for oral administration include tablets, capsules, granules, or pellets, which may eventually be coated with one of more functional and/or non-functional layers. Tablets are preferred dosage forms.

The invention also encompasses a tablet of candesartan cilexetil comprising the composition of the invention in an amount sufficient to provide an effective unit dose of candesartan cilexetil, and at least one auxiliary agent.

As mentioned above, candesartan cilexetil is an effective active substance for the treatment of hypertension. For management of hypertension, candesartan cilexetil may be orally administered in the form of tablets.

The effective unit dose of candesartan cilexetil may range from 1 mg to 50 mg per day, preferably from 2 mg to 30 mg per day.

The auxiliary agent may be selected from the group consisting of diluents, binders, lubricants, and disintegrating, antiadherent, colouring, sweetening, flavouring agents, and/or mixtures thereof.

Diluents are inert excipients that facilitate compression of pulverulent materials and provide resistance to tablets. Suitable diluents include corn starch, microcrystalline cellulose, powdered cellulose, silicified cellulose, lactose monohydrate, anhydrous lactose, mannitol, sorbitol, sucrose, fructose, dextrose, and/or mixtures thereof. Preferably corn starch and mannitol are used.

Binding agents are compounds that are able to provide cohesive properties to pulverulent material, in such a way that the fluidity of the composition is improved. The binding agent is selected from the group consisting of hydroxypropyl cellulose, hydroxypropylmethyl cellulose, hydroxyethyl cellulose, methylcellulose, polyvinylpyrrolidone, sodium carboxymethyl cellulose, calcium carboxymethyl cellulose, and/or mixtures thereof. Preferably hydroxypropyl cellulose is used.

Disintegrating agents are excipients that promote the rapid breakup of the tablet in an aqueous medium, as well as the rapid disintegration of the granule in order to release more quickly the active substance. Disintegrating agents may be selected from the group consisting of low-substituted hydroxypropyl cellulose, sodium carboxymethyl cellulose, calcium carboxymethyl cellulose, crospovidone, sodium croscarmellose, and/or mixtures thereof. Disintegrating agents may be incorporated into the intragranular phase and/or into the extragranular phase.

Lubricants and antiadherent agents are excipients that reduce interparticular friction and prevent adhesion of drug particles, and improve fluidity of granular or pulverulent compositions. Lubricants may be selected from the group consisting of talc, alkaline earth salts of stearic acid, specially magnesium and calcium stearate, stearic acid, glycerin palmitostearate, stearyl fumarate, and/or mixtures thereof. Colloidal silica is most preferred as an antiadherent agent.

The compositions of this invention may also contain sweetening and flavouring agents in order to provide acceptable organoleptic properties (flavour and taste) for patients. Suitable sweetening agents include sodium saccharin, aspartame, mannitol, xylitol, sucrose, sorbitol and ammonium glycyrrhizinate. Suitable flavouring agents include fruit and plant flavours, for example orange, anise, mint, etc.

Suitable colouring agents, which may be incorporated into the tablets of the invention, may be selected from those approved for oral use.

The tablets may be coated using conventional methods known to a person skilled in the art, as those described in Remington: The Science and Practice of Pharmacy, 20th Edition, Philadelphia, Lippincott, Williams & Wilkins, 2000 [ISBN 0 683 306472]. Among the film-forming agents which are used for coating the tablets, polyvinylpyrrolidone, hydroxypropylmethyl cellulose, hydroxypropyl cellulose, methyl cellulose, ethyl cellulose, hypromelose, solid polyethylenglycol, and polyvinyl alcohol may be included. Suitable auxiliary agents include pigments, plastifiers, and surfactants.

Information on the characteristics of auxiliary agents is described in reference handbooks available to those skilled in the art, for example in Handbook of Pharmaceutical Excipients, 4th Edition, London, Pharmaceutical Press, 2003 [ISBN 0 85369 472 9] wherein, in addition, the trade names of commercially available auxiliary agents are included.

In the solid pharmaceutical dosage forms of the invention, such as granules and tablets, the concentration of candesartan cilexetil usually ranges from 0.5% to 35% by weight on the total weight of the composition, preferably from 1% to 15%, and even more preferably from 2% to 10%. The concentration of the stabilizing agent may range from 0.1 % to 20% by weight on the total weight of the composition, more preferably from 0.5% to 10%, and even more preferably from 1 % to 5%.

The solid pharmaceutical dosage form (for example tablets) may include another active substance selected from the group consisting of diuretics, vasodilators and ACE inhibitors. Suitable diuretics include, for example, acetazolamide, alipamide, benzolamide, clopamide, furosemide and hydrochlorothiazide. Suitable vasodilators include, for example, felodipine, nicardipine and nifedipine. Suitable ACE inhibitors include, for example, perindopril, lisinopril and ramipril. A diuretic agent is preferred, more preferably hydrochlorothiazide.

In a preferred embodiment, the solid pharmaceutical dosage form of the invention is a tablet of candesartan cilexetil comprising:
a) from 1% to 15% by weight of candesartan cilexetil
b) from 1 % to 10% by weight of binder
c) from 1% to 6% by weight of stabilizing agent selected from the group consisting of diethylenglycol monoethyl ether, , DL-panthenol and potassium palmitate.
d) from 47% to 96.9% by weight of diluent,
e) from 0.1% to 2% by weight of lubricant, and
f) from 0% to 20% by weight of a diuretic active ingredient.

Preferably the binding agent is hydroxypropyl cellulose the diluent is selected from the group consisting of mannitol, lactose, corn starch and/or a mixture thereof and the lubricant is magnesium stearate.

A preferred embodiment encompasses a tablet comprising hydrochlorothiazide as diuretic active ingredient, and more preferably in a percentage from 6% to 10% w/w.

The weight percent refers to the total weight of the composition.

Surprisingly, solid pharmaceutical compositions of candesartan cilexetil containing a stabilizing agent selected from the abovementioned group have shown good stability in accelerated stability tests at 40°C and 75% relative humidity temperature, and also at 50°C temperature. Test results indicated that the content of degradation products in these compositions was significantly lower than the values obtained in compositions without stabilizing agent. On the other hand, this percentage in a similar composition but without stabilizing agent goes from 0.28% to 1.84% after 28 days at 50°C.

The following examples are given to illustrate the invention in a sufficiently complete manner.

### Examples

### Comparative Example 1 Preparation of tablets containing 4 mg of candesartan cilexetil and triethyl citrate as stabilizing agent

All the percentages indicated in the examples are weight percent of the total weight of the composition.

In a vessel, 15.4 g (3.1%) of candesartan cilexetil, 345.5 g (69.1%) of lactose and 77.5 g (15.5%) of corn starch were mixed.

The mixture was granulated with an aqueous solution of 40 g (8%) of hydroxypropyl cellulose and 20 g (4%) of triethyl citrate as stabilizing agent.

The resulting granulate was dried in a stove at a temperature of 50°C until humidity was lower than 3%.

The granules were sieved, and then 1.5 g (0.3%) of magnesium stearate were added and mixed.

The lubricated granules were compressed into 130-mg tablets by using 7-mm diameter punches on a compressing machine (Fette 1021i).

Tablets weighing 130 mg were obtained, each one containing 4 mg of candesartan cilexetil.

### Examples 2 to 8 Preparation of tablets containing 4 mg of candesartan cilexetil and different stabilizing agents

Using a procedure similar to that described in Example 1, 130 mg tablets containing 4 mg of candesartan cilexetil and the stabilizing agents shown in Table 1 were prepared.

**TABLE I**

| Example | Stabilizing agent | Amount (% w/w) |
|---|---|---|
| 2 Comparative | 2-octyldodecyl myristate | 4 |
| 3 Comparative | Isostearyl isostearate | 4 |
| 4 | Diethylenglycol monoethyl ether | 4 |
| 5 Comparative | Butyl stearate | 4 |
| 6 | DL-panthenol | 4 |
| 7 | Potassium palmitate | 4 |
| 8 Comparative | Triethyl citrate | 1 |

### Example 9 Preparation of tablets containing 8 mg of candesartan cilexetil, 12.5 mg of hydrochlorothiazide, and triethyl citrate as stabilizing agent

In a vessel, 24.2 g (4.8%) of candesartan cilexetil, 37.8 g (7.6%) of hydrochlorothiazide, 316.6 g (63.3%) of lactose and 72.4 g (14.5%) of corn starch were mixed.

The mixture was granulated with an aqueous solution of 37.2 g (7.5%) of hydroxypropyl cellulose and 10 g (2%) of triethyl citrate as stabilizing agent.

The resulting granulate was dried in a stove at a temperature of 50°C until humidity was lower than 3%.

The granules were sieved, and then 1.5 g (0.3%) of magnesium stearate were added and mixed.

The lubricated granules were compressed into 165-mg tablets by using 7-mm diameter punches on a compressing machine (Fette 1021i).

Tablets weighing 165 mg were obtained, each one containing 8 mg of candesartan cilexetil and 12.5 mg of hydrochlorothiazide.

### Comparative Example Preparation of tablets containing 4 mg of candesartan cilexetil but without stabilizing agent

Using a procedure similar to that described in Example 1, 130-mg tablets were prepared without the addition of stabilizing agents.

### Example 10 Stability test

The tablets as prepared according to Examples 1 to 8 and Comparative Example were subjected to an accelerated stability test in a stove under two different conditions:
- 40°C and 75% relative humidity (RH), and
- 50°C

HPLC analysis was used to determine the content of impurities in the tablets at the beginning of the test period and after standing for 14 days and 28 days under the abovementioned conditions.

The samples were prepared by placing 10 tablets in a 25-ml volumetric flask. About 3 ml of methanol were added, and the flask was immersed in an ultrasonic bath until completion of tablet disintegration. Then about 5 ml of acetonitrile were added, and the flask was immersed again in the ultrasonic bath for 15 minutes. The flask was brought to volume with acetonitrile and mixed. The obtained suspension was filtered, and the resulting solution was used for HPLC analysis in order to determine the content of impurities.

The HPLC equipment consisted of a Waters Alliance system with UV detection at 254 nm, L1-type column (according to USP specifications), at a temperature of 25°C, with a flow rate of 1 ml/min, and an injection volume of 10 µl.

The mobile phase was constituted of a mixture of solution A (v/v) (0.1% v/v aqueous buffer of trifluoroacetic acid) and acetonitrile.

During analysis the mobile phase had 45% of solution A and 55% of acetonitrile except during the 15-25 minute period, in which a mixture formed by 5% solution A and 95% acetonitrile was used.

Table II shows the results of the stability, expressed as a percentage of degradation products with respect to active substance found in the tablets at the beginning of test period and after storage at accelerated conditions:

**TABLE II**

| Example | Before test | After 14 days 40° C 75% RH | After 28 days 40° C 75% RH | After 14 days 50° C | After 28 days 50° C |
|---|---|---|---|---|---|
| 1 Comparative | 0.30 | 0.31 | 0.31 | 0.37 | 0.45 |
| 2 Comparative | 0.26 | 0.51 | 0.41 | 0.53 | 0.57 |
| 3 Comparative | 0.25 | 0.39 | 0.39 | 0.50 | 0.56 |
| 4 | 0.24 | 0.31 | 0.26 | 0.40 | 0.40 |
| 5 Comparative | 0.24 | 0.27 | 0.34 | 0.33 | 047 |
| 6 | 0.26 | 0.32 | 0.42 | 0.45 | 0.69 |
| 7 | 0.28 | 0.39 | 0.54 | 0.57 | 1.00 |
| 8 Comparative | 0.22 | 0.28 | 0.33 | 0.38 | 0.48 |
| Comparative | 0.28 | 0.83 | 1.13 | 1.39 | 1.84 |

One can observe that the content of degradation products in the samples of the tablets prepared from the compositions of the invention is significantly lower than that in the sample of tablets prepared from formulations that do not contain a stabilizing agent.

Therefore, it can be concluded that the compositions of candesartan cilexetil of the present invention show good stability of the active ingredient.

## Claims

1. A solid pharmaceutical composition for oral use comprising:
a) a pharmaceutically effective amount of candesartan cilexetil, and
b) a stabilizing agent selected from the group consisting of:
i) ethers of C₁-C₄ monohydroxy alcohols and C₂-C₉ polyhydroxy alcohols,
ii) saturated fatty acid alkaline salts, and
iii) panthenol.

2. The composition according to claim 1 **characterized in that** the stabilizing agent is selected from ethers of C₁-C₄ monohydroxy alcohols and C₂-C₉ polyhydroxy alcohols.

3. The composition according to claim 2 **characterized in that** the stabilizing agent is diethylenglycol ethyl ether.

4. A process for preparing a composition according to any of claims 1 to 3 comprising mixing candesartan cilexetil with the stabilizing agent, and moulding the mixture.

5. Use of a composition according to any of claims 1 to 3 for the preparation of solid oral dosage forms of candesartan cilexetil.

6. A tablet of candesartan cilexetil comprising a composition according to any of claims 1 to 3 in an amount sufficient to provide an effective unit dose of candesartan cilexetil, and at least one auxiliary agent.

7. The tablet according to claim 6 **characterized in that** the content of the stabilizing agent is from 0.5% to 10% by weight on the total weight of the composition.

8. The tablet according to claim 7 **characterized in that** the content of the stabilizing agent is from 1% to 5% by weight on the total weight of the composition.

9. The tablet according to any of claims 6 to 8 **characterized in that** the content of candesartan cilexetil is from 1 % to 15% by weight on the total weight of the composition.

10. The tablet according to claim 9 **characterized in that** the content of candesartan cilexetil is from 2% to 10% by weight on the total weight of the composition.

11. The tablet according to any of claims 6 to 10 **characterized in that** it comprises another active ingredient selected from the group consisting of diuretics, vasodilators, and ACE inhibitors.

12. A tablet of candesartan cilexetil **characterized in that** it comprises:
a) from 1% to 15% by weight of candesartan cilexetil
b) from 1 % to 10% by weight of binder
c) from 1% to 6% by weight of stabilizing agent selected from the group consisting of diethylenglycol monoethyl ether, DL-panthenol and potassium palmitate.
d) from 47% to 96,9% by weight of diluent,
e) from 0.1 % to 2% by weight of lubricant, and
f) from 0% to 20% of a diuretic active substance.

13. The tablet according to any of claims 11 to 12 **characterized in that** it comprises hydrochlorothiazide.

## Patentansprüche

1. Feste pharmazeutische Zusammensetzung zur oralen Verabreichung umfassend:
a) eine pharmazeutisch wirksame Menge von Candesartancilexetil, und
b) ein Stabilisierungsmittel ausgewählt aus der Gruppe bestehend aus:
i) Ethern aus C₁-C₄ Monohydroxyalkoholen und C₂-C₉ Polyhydroxyalkoholen,
ii) Alkalisalzen aus gesättigten Fettsäuren, und
iii) Panthenol.

2. Zusammensetzung nach Anspruch 1 **dadurch gekennzeichnet, dass** das Stabilisierungsmittel ausgewählt ist aus Ethern aus C₁-C₄ Monohydroxyalkoholen und C₂-C₉ Polyhydroxyalkoholen.

3. Zusammensetzung nach Anspruch 2 **dadurch gekennzeichnet, dass** das Stabilisierungsmittel Diethylenglycolethylether ist.

4. Verfahren zur Herstellung einer Zusammensetzung nach einem der Ansprüche 1 bis 3 umfassend das Mischen von Candesartancilexetil mit dem Stabilisierungsmittel und die Formung der Mischung.

5. Verwendung einer Zusammensetzung nach einem der Ansprüche 1 bis 3 zur Herstellung von festen oralen Darreichungsformen von Candesartancilexetil.

6. Candesartancilexetil-Tablette umfassend eine Zusammensetzung nach einem der Ansprüche 1 bis 3 in einer Menge, die ausreicht, um eine wirksame Einzeldose von Candesartancilexetil zu erhalten, und mindestens ein Hilfsmittel.

7. Tablette nach Anspruch 6 **dadurch gekennzeichnet, dass** der Gehalt vom Stabilisierungsmittel von 0,5 Gew.-% bis 10 Gew.-% bezüglich des Gesamtgewichts der Zusammensetzung reicht.

8. Tablette nach Anspruch 7 **dadurch gekennzeichnet, dass** der Gehalt vom Stabilisierungsmittel von 1 Gew.-% bis 5 Gew.-% bezüglich des Gesamtgewichts der Zusammensetzung reicht.

9. Tablette nach einem der Ansprüche 6 bis 8 **dadurch gekennzeichnet, dass** der Gehalt von Candesartancilexetil von 1 Gew.-% bis 15 Gew.-% bezüglich des Gesamtgewichts der Zusammensetzung reicht.

10. Tablette nach Anspruch 9 **dadurch gekennzeichnet, dass** der Gehalt von Candesartancilexetil von 2 Gew.-% bis 10 Gew.-% bezüglich des Gesamtgewichts der Zusammensetzung reicht.

11. Tablette nach einem der Ansprüche 6 bis 10 **dadurch gekennzeichnet, dass** sie einen weiteren Wirkstoff umfasst ausgewählt aus der Gruppe bestehend aus Diuretika, Vasodilatatoren und ACE-Hemmern.

12. Candesartancilexetil-Tablette **dadurch gekennzeichnet, dass** sie folgendes umfasst:
a) von 1 Gew.-% bis 15 Gew.-% Candesartancilexetil
b) von 1 Gew.-% bis 10 Gew.-% eines Bindemittels
c) von 1 Gew.-% bis 6 Gew.-% eines Stabilisierungsmittels ausgewählt aus der Gruppe bestehend aus Diethylenglycolmonoethylether, DL-Panthenol und Kaliumpalmitat.
d) von 47 Gew.-% bis 96,9 Gew.-% eines Verdünnungsmittels,
e) von 0,1 Gew.-% bis 2 Gew.-% eines Gleitmittels, und
f) von 0 Gew.-% bis 20 Gew.-% eines diuretischen Wirkstoffs.

13. Tablette nach einem der Ansprüche 11 bis 12 **dadurch gekennzeichnet, dass** sie Hydrochlorothiazid umfasst.

## Revendications

1. Composition pharmaceutique solide pour une utilisation orale comprenant:
a) une quantité pharmaceutiquement efficace de candésartan cilexétil, et
b) un agent de stabilisation choisi dans le group constitué:
i) des éthers d'alcools monohydroxyliques en C₁ à C₄ et d'alcools polyhydroxyliques en C₂ à C₉,
ii) des sels alcalins d'acides gras saturés, et
iii) du panthénol.

2. Composition selon la revendication 1 **caractérisée en ce que** l'agent de stabilisation est choisi parmi les éthers d'alcools monohydroxyliques en C₁ à C₄ et d'alcools polyhydroxyliques en C₂ à C₉.

3. Composition selon la revendication 2 **caractérisée en ce que** l'agent de stabilisation est le diéthylène glycol éthyl éther.

4. Procédé de préparation d'une composition selon l'une quelconque des revendications 1 à 3 comprenant mélanger du candésartan cilexétil avec l'agent de stabilisation, et le moulage du mélange.

5. Utilisation d'une composition selon l'une quelconque des revendications 1 à 3 pour la préparation de formes de dosage oral solides de candésartan cilexétil.

6. Comprimé de candésartan cilexétil comprenant une composition selon l'une quelconque des revendications 1 à 3 dans une quantité suffisante pour donner une dose unitaire de candésartan cilexétil, et au moins un agent auxiliaire.

7. Comprimé selon la revendication 6 **caractérisé en ce que** la teneur en agent de stabilisation va de 0,5% à 10% en poids par rapport au poids total de la composition.

8. Comprimé selon la revendication 7 **caractérisé en ce que** la teneur en agent de stabilisation va de 1 % à 5% en poids par rapport au poids total de la composition.

9. Comprimé selon l'une quelconque des revendications 6 à 8 **caractérisé en ce que** la teneur en candésartan cilexétil va de 1% à 15% en poids par rapport au poids total de la composition.

10. Comprimé selon la revendication 9 **caractérisé en ce que** la teneur en candésartan cilexétil va de 2% à 10% par rapport au poids total de la composition.

11. Comprimé selon l'une quelconque des revendications 6 à 10 **caractérisé en ce qu'**il comprend un autre ingrédient actif choisi dans le groupe constitué des diurétiques, des vasodilatateurs, et des inhibiteurs de l'ECA.

12. Comprimé de candésartan cilexétil **caractérisé en ce qu'**il comprend:
a) de 1% à 15% en poids de candésartan cilexétil
b) de 1% à 10% en poids d'un liant
c) de 1% à 6% en poids d'un agent de stabilisation choisi dans le group constitué du diéthylène glycol monoéthyl éther, du DL-panthénol et du palmitate de potassium.
d) de 47% à 96,9% en poids d'un diluant,
e) de 0,1% à 2% en poids d'un lubrifiant, et
f) de 0% à 20% d'une substance active diurétique.

13. Comprimé selon l'une quelconque des revendications 11 à 12 **caractérisé en ce qu'**il comprend de l'hydrochlorothiazide.
